# EUROPEAN PATENT SPECIFICATION

(11) **EP 2 148 614 B1**
(45) Date of publication and mention of the grant of the patent: **11.04.2012**
(21) Application number: 08737203.3
(22) Date of filing: 21.04.2008
(51) Int. Cl.: A61B 5/053, A61B 18/20

(54) **APPARATUS FOR DETECTING THE PRESENCE OF SKIN**
GERÄT ZUM NACHWEIS DER ANWESENHEIT VON HAUT
APPAREIL POUR DÉTECTER LA PRÉSENCE DE PEAU

(30) Priority: 20.04.2007 DK 200700581
(43) Date of publication of application: 03.02.2010
(73) Proprietor: Cyden Ltd., Swansea SA1 8PH (GB)
(72) Inventor: FISHER, Simon James, Swansea West Glamorgan SA4 9LB (GB)
(74) Representative: Austin, Hedley William
(86) International application number: PCT/GB2008/050278
(87) International publication number: WO 2008/129324

(56) References cited:
- EP-A- 1 433 499
- WO-A-2006/038168
- US-A1- 2004 230 260
- US-A1- 2006 149 343

## Description

The present invention relates to apparatus for detecting the presence of skin, typically for use as a safety device to ensure that light therapy devices are not used where there is no target area of skin present. The invention also encompasses a method for detecting of the presence of skin suitable for use in controlling operation of such light therapy devices, both for therapeutic purposes and for cosmetic purposes).

Various types of light therapy are known. For example, intense-pulsed-light (IPL) is used for local treatment of various skin conditions and to influence non-desired hair growth.

It is important to ensure that the light is directed at the area of skin to be treated, and not for example, at more delicate body parts (such as the eyes) and not used where skin is not detected.

US2004/0167502 A1 discloses a device and method for sensing the presence of human skin by evaluating the spectral remittance of electromagnetic radiation from the surface in question. Such a device requires complicated circuitry and optical control, separate from the optical control needed in apparatus for dermatological treatment. It is an object of the invention to provide simplified apparatus for this purpose.

Apparatus for detecting the presence of skin, of known type comprises
a) a set of probes each having tips arranged to simultaneously touch the skin and define a predetermined pattern on the skin, at least one of said probes (hereinafter referred to as a transmitter probe) being arranged to transmit a pulsed electrical signal and at least one of said probes (hereinafter referred to as a receiver probe) being arranged to receive said transmitted electrical signal;
b) signal detector means for detecting the or each received electrical signal;
c) means for comparing a numerical value obtained from at least one detected signal from said signal detector means with at least one predetermined numerical value d) means for providing an output when the value obtained from the detected signal differs from the predetermined numerical value by more than a predetermined amount.

A skin sensor having these features is known, for example, from WO2006/038168. Amongst the embodiments disclosed in the latter document is an arrangement using two sensors in which an electric voltage is passed between the sensors and the resulting current between the two sensors is measured.

According to the invention the receiver probe is the same as the transmitter probe, such that the probe both transmits a pulsed electrical signal and receives the transmitted electrical signal. The apparatus may take measurements based on capacitance (such as elapsed time to reach a predetermined voltage). It is preferred that all of the set of probes are both transmitter probes and receiver probes.

In an advantageous embodiment of the invention, the tips of the probes are fixed to a support, such that the tips together define a two dimensional pattern, such as a rectangle, in which all the tips are intended to be in contact with the skin. This pattern preferably defines the perimeter of an area that is slightly larger than the area of an aperture of an intense-pulsed-light device, such as a discharge lamp, which is to be contacted with the skin. Such a pattern is typically a rectangle measuring about 10 to 15 mm x 20 to 30 mm. An arrangement of the nature just described ensures that the aperture is not obscured by the probes.

The probes themselves are preferably elongate pins, typically having a diameter of about 2 to 3 mm.

Because dry skin, oily skin and gel-covered skin all have different electrical properties, including conductance and capacitance, it is preferred that the predetermined amount is sufficiently distinct from the predetermined value in order to provide a margin of error.

For example, if the predetermined value is voltage, it is preferred that the minimum of the range be above the minimum known for skin. If the predetermined value relates to capacitance, the predetermined value is preferably based on elapsed time to reach a threshold voltage which, in turn will depend on the capacitance of the skin contacted by the probe.

The apparatus according to the invention may be used for the detection of the presence of skin in an automated decision stage of a microprocessor to activate an intense-pulsed-light unit when pre-determined input conditions are fulfilled (that is when skin having parameters within a predetermined range is detected).

An advantageous use of the apparatus is to automatically determine whether the parameters measured indicate that a gel-like substance has been applied to the skin. In this case, the firing of the intense-pulsed-light is only permitted when such gel-coated skin is detected.

The output provided when the values are outside a predetermined range may comprise an alarm, which can typically be one or more of an audio signal, a visible signal or a movement signal (such as a vibration or other movement of the apparatus). Alternatively, the output may be a feedback signal arranged to cause an intense-pulsed-light source to cut out when values outside the predetermined range are detected.

A further aspect of the invention relates to a method for controlling the operation of an intense-pulsed-light source, which comprises applying a set of probes each having tips simultaneously touching a test surface so as to define a predetermined pattern on the test surface, detecting signals between the probes when the probes contact the test surface, comparing a value of the detected signals from the or each signal detector with at least one predetermined value transmitting a pulsed electrical signal from at least one of the probes; and providing an output when the compared value is outside a predetermined range, which output either prevents or permits the operation of the light source.

According to the invention, the signal is a pulsed electrical signal from at least one of the probes which probe further receives the transmitted electrical signal to provide the output to either prevent or permit the operation of the light source.

Preferred embodiments of the present invention will be further described, by way of example only, with reference to the accompanying drawings, in which
Figure 1 is a schematic representation of four probes used in apparatus according to the invention;
Figure 2 is a circuit diagram of apparatus according to a second embodiment of the invention;
Figure 3 is a block diagram showing preferred aspects of operation of apparatus according to the invention; and,

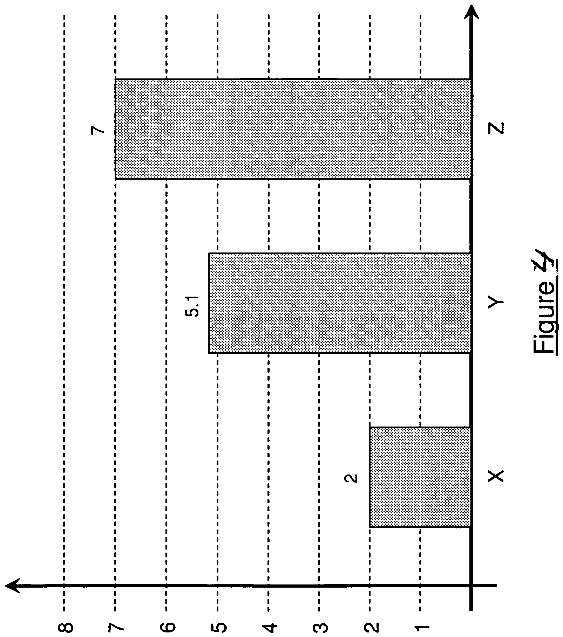

Figure 4 is graph showing typical threshold values for use in apparatus according to the present invention.

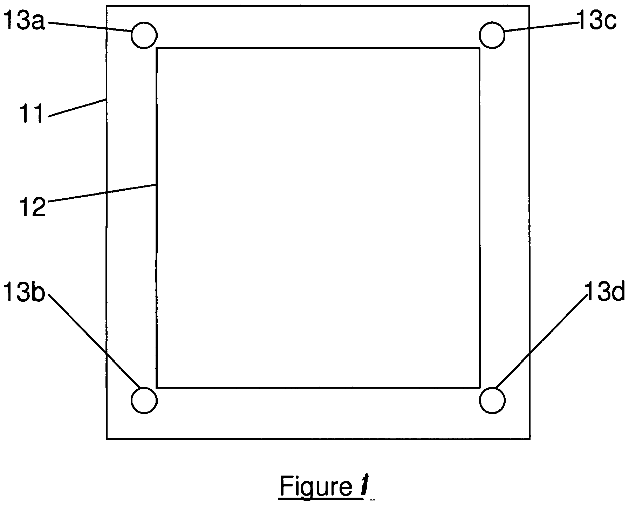

Referring to Figure 1, there is shown a front view of apparatus that contains a discharge tube (not shown) for generating intense-pulsed-light. The tube is protected by a glass block 11, which is within a substantially rectangular aperture 12 of a frame, with the probes 13a to 13d being arranged each with an insulating support and positioned just outside the corners of the aperture 12.

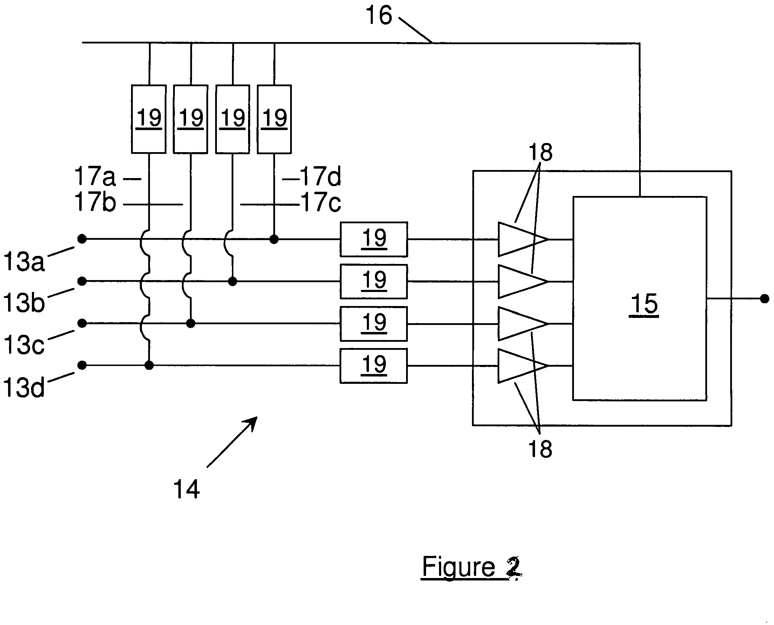

Exemplary control circuitry, which controls the firing of the tube (not shown) in accordance with the invention, is shown in Figure 2, and includes probes 13a to 13d corresponding to the reference numerals used in Figure 1).

The control circuit 14 shown in Figure 2 comprises a processing unit 15 which receives directly as input from a power rail 16, a continuous train of periodic pulses of duration T. This input is used to establish a time reference frame for each of the subsequent inputs to the processing unit 15 via circuit paths 17a to 17d. Each circuit path 17a to 17d comprises a respective comparator 18 which separately receives as input, a voltage from the power rail 16. Each of the comparators 18 is arranged to generate a respective output to the processing unit 15 when the voltage at the respective input reaches a predetermined threshold, for example about 3.8V.

The voltage input to each comparator 18 is governed by a pair of resistors 19 each typically being about 100kΩ, arranged in a series configuration with the respective comparator 18. Each of the four probes 13a to 13d is separately connected to a respective circuit path 17a to 17d at a position intermediate the respective pairs of series resistors 19. The probes 13a to 13d in the embodiment of Figure 2 may be arranged in a manner such as that described above with reference to Figure 1, and are each arranged to transmit and receive an electrical signal.

The distal end of each probe 13a to 13d may be initially located in free space (in contact with air) and will thus generate a small capacitance. Accordingly, the application of a voltage pulse to the power rail 16 will cause the voltage input to the respective comparator 18 to increase to the threshold voltage over a time interval t₁. If the probes 13a to 13d are all in contact with skin, then this will act to increase the effective capacitance of the probes 13a to 13d thereby causing an increase in the interval t₁.

In use, a periodic train of voltage pulses, each of magnitude approximately 5V and duration up to 100µs is continuously applied to the power rail 16. At a time corresponding to the leading edge of each voltage pulse on the power rail 16, the voltage input to each comparator 18 will begin to increase. The rate of increase of the voltage input to each comparator 18 will depend upon whether the respective probe 13a to 13d is in free space or coupled to skin. In the latter case, the rate of increase of the voltage input to each comparator 18 will further depend on the condition of the skin and on whether a coupling gel has been applied to the skin.

Probes 13a to 13d will, when coupled to the skin, cause the voltage input to the respective comparator 18 to increase more slowly than for those comparators for which the probes are arranged in free space. Moreover, it is found that the application of a coupling gel to the skin further reduces the rate of voltage rise input to the respective comparator 18.

The processing unit 15 only outputs a signal to cause the discharge lamp (not shown) to generate a pulse of intense light when it is determined that all four probes 13a to 13d are in contact with skin. Accordingly, the time for the comparators 18 to change output state is monitored and only if the time taken for each comparator 18 to change output state is above a threshold time, as determined from the leading edge of a selected voltage pulse on the power rail 16, will the processing unit 15 instruct the firing of the discharge lamp (not shown). In this respect, it is not necessary for each comparator 18 to change output state. If the output from each and every comparator 18 does not change state due to an excessively long rise time, then this is further taken to indicate that the respective probe(s) 13a to 13d is/are in contact with the skin.

The continuous application of voltage pulses to the power rail 16 thus provides for a continuous verification of whether the probes 13a to 13d are in contact with skin, and thus the circuit 14 will inhibit the firing of the discharge lamp (not shown) if at least one of the probes 13a to 13d breaks or loses contact with the skin.

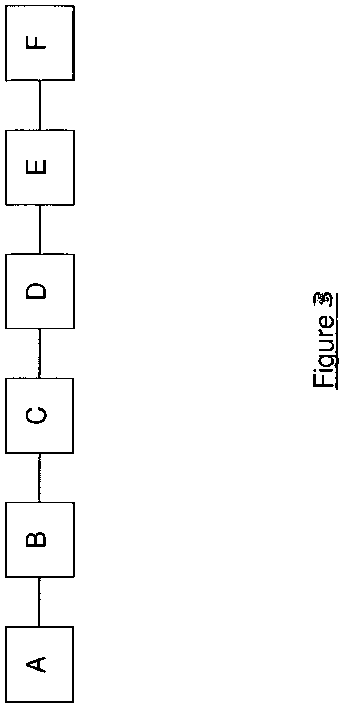

3 Referring to Figure 3, there is shown an exemplary flow chart, showing the sequence of operations or steps in the method of controlling the operation of an intense-pulsed-light source according to the present invention. Block A represents the first step, namely the application of gel to the skin. Block B represents the second step, namely the application of an array of probes 2 to 5 or 13a to 13d, to the skin.

A pulsed electrical signal is sent through the probes 2 to 5, or 13a to 13d in step C and the time to reach the threshold voltage for each probe 2 to 5, or 13a to 13d is assessed in step D. The time is compared to a stored threshold value in step E; when the time is found to be greater than the stored threshold value in step F, the processing unit 10 or 15 outputs a control signal to permit a discharge lamp (not shown) to fire. Conversely, when the time is determined to be less that the stored threshold value in step F, the firing of the discharge lamp (not shown) is inhibited.

The stored threshold value chosen will depend to some extent on the frequency of the applied voltage pulse. Accordingly, it is preferably to normalise the time. Normalised time values of about 5.1 were obtained using apparatus according to the invention (with the probes 13a to 13d being both transmitters and receivers) with oily skin; about 5.4 with degreased skin; about 6.3 for sweaty skin and over 7.0 for gel-coated skin - depending on the nature of the skin to which the gel was applied.

The stored time, below which firing is not permitted, may therefore be based on the above values, such as about 4. In that case, the firing will be permitted even when the probes 13a to 13d are not in contact with gel-coated skin. Alternatively, the stored output time below which firing is not permitted, may be set at about 7; in which case the firing will only be permitted when the probes 13a to 13d are in contact with gel-coated skin.

Figure 5 provides a graphical comparison of the normalised time taken for the comparators to change state, when the probes 13a to 13d are arranged in free space - as indicated by column X, when the probes 13a to13d are arranged in contact with skin - as indicated by column Y, and when the probes are arranged in contact with skin to which a gel has been applied - as indicated by column Z.

If the normalised time is less than a first threshold (column Y), this is taken to be indicative of the absence of skin, whereas if it exceeds a second threshold this is taken to be indicative of the presence of gel-coated skin (column Z). An intermediate value corresponds to a value measured for oily skin. If the detected value is below the first threshold, an alarm is triggered or a feedback signal is provided to prevent inadvertent operation of an intense-pulsed-light source.

From the foregoing therefore, it is evident that the apparatus and method of the present invention can prevent inadvertent operation of an intense-pulsed-light source when, for example one or more of the tips of the probes fail to contact skin, or for example when they contact another part of a body, such as an eye or mucous membrane.

## Claims

1. Apparatus for detecting the presence of skin, which apparatus comprises
a) a set of probes (2,3,4,5; 13a, 13b, 13c, 13d) each probe of the set of probes having a tip, the tips of each of said probes being arranged to simultaneously touch the skin and define a predetermined pattern on the skin;
b) signal detector means for detecting at least one electrical signal transmitted through skin when said probes touch the skin;
c) comparison means (18) for comparing a numerical value obtained from at least one detected signal from said signal detector means with at least one predetermined numerical value; and
d) means (15) for providing an output when said value obtained from the detected signal differs from said predetermined numerical value by more than a predetermined amount, **characterised in that** at least one of said probes is arranged both to transmit a pulsed electrical signal through said skin and to receive said electrical signal transmitted through skin.

2. Apparatus according to claim 1, wherein the predetermined pattern comprises a rectangle (12) defined by the tips of said probes.

3. Apparatus according to claim 1 or 2, wherein the probes comprise elongate pins each having a diameter of 2 to 3 mm.

4. Apparatus according to any preceding claim, wherein all of the set of probes are arranged both to transmit and receive said electrical signal.

5. Apparatus according to any of claims 1 to 4, in combination with an intense pulsed light source.

6. Apparatus according to claim 5, wherein said output comprises a feedback control for preventing operation of said light source.

7. A method of controlling operation of an intense pulsed light source, which method comprises the use of a set of probes, each probe of the set of probes comprising a tip, the method comprising applying (B) the tips simultaneously to a test surface so that the tips define a predetermined pattern on the test surface, and detecting signals between said probes when said probes contact the test surface,
comparing (E) a value of said detected signals with at least one predetermined value (F); and
providing an output when a resultant compared value is outside a predetermined range, **characterised in that** the signal is a pulsed electrical signal from at least one of the probes which probe further receives said transmitted electrical signal to provide said output to either prevent or permit said operation of said light source.

8. A method according to claim 7, in which there is employed apparatus according to any of claims 1 to 6.

## Patentansprüche

1. Vorrichtung zum Erfassen des Vorhandenseins von Haut, wobei die Vorrichtung umfasst:
a) einen Sondensatz (2, 3, 4, 5; 13a, 13b, 13c, 13d), wobei jede Sonde des Sondensatzes eine Spitze hat, wobei die Spitzen jeder der Sonden so angeordnet sind, dass sie gleichzeitig die Haut berühren und ein vorbestimmtes Muster auf der Haut definieren;
b) ein Signaldetektormittel zum Erfassen mindestens eines elektrischen Signals, das durch die Haut übertragen wird, wenn die Sonden die Haut berühren;
c) ein Vergleichsmittel (18) zum Vergleichen eines numerischen Wertes, der von mindestens einem vom Signaldetektormittel erfassten Signal erhalten wird, mit mindestens einem vorbestimmten numerischen Wert; und
d) ein Mittel (15) zum Bereitstellen eines Ausgangs, wenn sich der Wert, der vom erfassten Signal erhalten wird, von dem vorbestimmten numerischen Wert um mehr als ein vorbestimmtes Maß unterscheidet, **dadurch gekennzeichnet, dass** mindestens eine der Sonden so angeordnet ist, dass sie sowohl ein gepulstes elektrisches Signal durch die Haut überträgt wie auch das durch die Haut übertragene Signal empfängt.

2. Vorrichtung nach Anspruch 1, wobei das vorbestimmte Muster ein Rechteck (12) umfasst, das durch die Spitzen der Sonden definiert ist.

3. Vorrichtung nach Anspruch 1 oder 2, wobei die Sonden längliche Stifte mit jeweils einem Durchmesser von 2 bis 3 mm umfassen.

4. Vorrichtung nach einem der vorangehenden Ansprüche, wobei alle aus dem Sondensatz sowohl zum Übertragen wie auch Empfangen des elektrischen Signals angeordnet sind.

5. Vorrichtung nach einem der Ansprüche 1 bis 4 in Kombination mit einer intensiven gepulsten Lichtquelle.

6. Vorrichtung nach Anspruch 5, wobei der Ausgang eine Rückkopplungssteuerung zum Verhindern des Betriebs der Lichtquelle umfasst.

7. Verfahren zum Steuern des Betriebs einer intensiven gepulsten Lichtquelle, wobei das Verfahren die Verwendung eines Sondensatzes umfasst, wobei jede Sonde des Sondensatzes eine Spitze umfasst, wobei das Verfahren das gleichzeitige Aufbringen (B) der Spitzen auf einer Testoberfläche, so dass die Spitzen ein vorbestimmtes Muster auf der Testoberfläche definieren, sowie das Erfassen von Signalen zwischen den Sonden, wenn die Sonden mit der Testoberfläche in Kontakt sind, umfasst, das Vergleichen (E) eines Wertes der erfassten Signale mit mindestens einem vorbestimmten Wert (F); und
das Bereitstellen eines Ausgangs, wenn ein erhaltener Vergleichswert außerhalb eines vorbestimmten Bereichs liegt, **dadurch gekennzeichnet, dass** das Signal ein gepulstes elektrisches Signal von mindestens einer der Sonden ist, wobei die Sonde des Weiteren das übertragene elektrische Signal empfängt, um den Ausgang bereitzustellen, so dass der Betrieb der Lichtquelle entweder verhindert oder ermöglicht wird.

8. Verfahren nach Anspruch 7, wobei eine Vorrichtung nach einem der Ansprüche 1 bis 6 verwendet wird.

## Revendications

1. Appareil de détection de la présence de peau, ledit appareil comprenant :
a) un ensemble de sondes (2, 3, 4, 5 ; 13a, 13b, 13c, 13d) chaque sonde de l'ensemble de sondes ayant une extrémité, les extrémités de chacune desdites sondes étant configurées de manière à simultanément toucher la peau et définir un motif prédéterminé sur la peau ;
b) un moyen de détection de signal pour détecter au moins un signal électrique transmis à travers la peau lorsque lesdites sondes touchent la peau ;
c) un moyen de comparaison (18) pour comparer une valeur numérique obtenue à partir d'au moins un signal détecté par ledit moyen de détection de signal avec au moins une valeur numérique prédéterminée ; et
d) un moyen (15) pour fournir une sortie lorsque la différence entre ladite valeur obtenue à partir du signal détecté et ladite valeur numérique prédéterminée est supérieure à une quantité prédéterminée, **caractérisé en ce qu'**au moins l'une desdites sondes est configurée à la fois pour transmettre un signal électrique pulsé à travers ladite peau et pour recevoir ledit signal électrique transmis à travers la peau.

2. Appareil selon la revendication 1, dans lequel le motif prédéterminé comprend un rectangle (12) défini par les extrémités desdites sondes.

3. Appareil selon la revendication 1 ou 2, dans lequel les sondes comprennent des tiges allongées ayant chacune un diamètre de 2 à 3 mm.

4. Appareil selon l'une quelconque des revendications précédentes, dans lequel la totalité des sondes de l'ensemble sont configurées pour transmettre et recevoir ledit signal électrique.

5. Appareil selon l'une quelconque des revendications 1 à 4, en combinaison avec une source de lumière pulsée intense.

6. Appareil selon la revendication 5, dans lequel ladite sortie comprend une commande à rétroaction pour empêcher le fonctionnement de ladite source de lumière.

7. Procédé de commande du fonctionnement d'une source de lumière pulsée intense, ledit procédé comprenant l'utilisation d'un ensemble de sondes, chaque sonde de l'ensemble de sondes comprenant une extrémité, le procédé comprenant l'application (B) des extrémités simultanément à une surface de test pour que les extrémités définissent un motif prédéterminé sur la surface de test, et la détection des signaux entre lesdites sondes lorsque lesdites sondes contactent la surface de test,
la comparaison (E) d'une valeur desdits signaux détectés à au moins une valeur prédéterminée (F) ; et
la fourniture d'une sortie lorsqu'une valeur comparée obtenue se trouve hors d'une plage prédéterminée, **caractérisé en ce que** le signal est un signal électrique pulsé d'au moins l'une des sondes, ladite sonde recevant en outre ledit signal électrique transmis pour fournir ladite sortie pour soit empêcher soit permettre ledit fonctionnement de ladite source de lumière.

8. Procédé selon la revendication 7, dans lequel est utilisé un appareil selon l'une quelconque des revendications 1 à 6.
